# EUROPEAN PATENT APPLICATION

(11) **EP 2 371 943 A1**
(43) Date of publication of application: **05.10.2011**
(21) Application number: 09834613.3
(22) Date of filing: 23.10.2009
(51) Int. Cl.: C12N 5/07, C12N 1/02, B04B 11/04, B04B 15/12

(54) **METHOD FOR WASHING CELLS**

(30) Priority: 25.12.2008 JP 2008331031
(71) Applicant: Olympus Corporation, Shibuya-ku Tokyo 151-0072 (JP); Cytori Therapeutics, Inc., San Diego, California 92121 (US)
(72) Inventor: TSUCHIDA, Hiroki, Tokyo 151-0072 (JP); KARASAWA, Chisato, Tokyo 151-0072 (JP); YOSHIDA, Kazuhiro, Tokyo 151-0072 (JP); SHANAHAN, Robert K., Carlsbad, California 92009 (US); FORNACE, Lucas V., La Jolla, California 92037 (US)
(74) Representative: Schicker, Silvia
(86) International application number: PCT/JP2009/068237
(87) International publication number: WO 2010/073808

(57) **Abstract**

A cell cluster from which unwanted components are removed by efficiently washing the cell cluster is collected. Provided is a cell washing method including a centrifuging step (S1) of collecting a cell suspension in a centrifuge container having a washing fluid discharge port opposing a bottom part and centrifugally separating cells at the bottom part by rotating in such a manner that the bottom part faces outward in the radial direction; a supernatant ejecting step (S2) of ejecting a supernatant centrifugally separated in the centrifuging step (S1); and a washing step (S3) of discharging the washing fluid from the discharge port while rotating the centrifuge container while ejecting the supernatant in the supernatant ejecting step (S2).

## Description

### {Technical Field}

The present invention relates to a cell washing method.

### {Background Art}

A known centrifuge apparatus in the related art separates components contained in a cell suspension by specific gravity by rotating a centrifuge container, which holds a cell suspension acquired by breaking down fat tissue and isolating fat-derived cells, around an axis away from the centrifuge container (refer to PTL 1).
The centrifuge container is substantially cylindrical with one end closed and is rotated in such a manner that the closed end faces outward in the radial direction to move components having large specific weights toward the closed end so that they are arranged in descending order of specific weight from the closed end side.

### {Citation List}

### {Patent Literature}

### {PTL 1}

PCT International Publication No. WO 2005/012480

### {Summary of Invention}

### {Technical Problem}

However, there is a disadvantage in that a cell cluster separated at a bottom part of a centrifuge container by centrifugally separating a cell suspension is packed into a solid pellet shape by a centrifugal force. That is, when the cell cluster is formed into a pellet shape, it becomes difficult to take out the cell cluster from the centrifuge container by suction after centrifugation. Sometimes a cell cluster may be formed into a pellet shape in which unwanted components, such as proteolytic enzymes and fat, remain trapped during centrifugation, and it is difficult to remove such unwanted components.

The present invention has been made in view of the circumstances described above, and an object thereof is to provide a cell washing method that can efficiently wash a cell cluster and collect a cell cluster with unwanted components removed therefrom.

### {Solution to Problem}

To achieve the object described above, the present invention provides the following solutions.
The present invention provides a cell washing method including a centrifuging step of collecting a cell suspension in a centrifuge container having a washing fluid discharge port opposing a bottom part and centrifugally separating cells at the bottom part by rotating in such a manner that the bottom part faces outward in the radial direction; a supernatant ejecting step of ejecting a supernatant centrifugally separated in the centrifuging step; and a washing step of discharging washing fluid from the discharge port while rotating the centrifuge container while ejecting the supernatant in the supernatant ejecting step.

According to the present invention, in the centrifuging step, the components contained in the cell suspension are separated by specific gravity by rotating the centrifuge container in such a manner that the bottom part of the centrifuge container holding the cell suspension faces outward in the radial direction. In such a case, a large cell cluster having a higher specific gravity is collected in the lowest layer at the bottom part of the centrifuge container, and a supernatant, such as plasma, is collected in the layer thereabove. The cell cluster is packed into a solid pellet shape by a centrifugal force.

In this state, by disposing the centrifuge container along the vertical direction such that the bottom part faces downward and by sucking the supernatant in the supernatant ejecting step, the supernatant can be ejected outside the container while the cell cluster pellet is left at the bottom part of the centrifuge container.
In this state, in the washing step, by discharging the washing fluid into the centrifuge container from the discharge port disposed at the bottom part of the centrifuge container while rotating the centrifuge container, the discharged washing fluid is sprayed on the pellet-shaped cell cluster packed at the bottom part of the centrifuge container, and the pellet-shaped cell cluster is broken down and scattered. In this way, a cell cluster in which unwanted components are trapped during centrifugation can be broken down to release the unwanted components into the washing fluid.

In the present invention, the centrifuging step and the supernatant ejecting step may be carried out again after the cells are washed in the washing step.
In this way, the cell suspension acquired by washing the cells in the washing step is centrifugally separated again in the centrifuging step and is separated into a pellet-shaped cell cluster and a supernatant. At this time, since the unwanted components are released into the supernatant, a cell cluster having a reduced concentration of unwanted components trapped inside can be obtained by carrying out the ejecting

### step again.

### {Advantageous Effects of Invention}

The present invention is advantageous in that a cell cluster in which unwanted components are removed therefrom by efficiently washing the cell cluster can be collected.

### {Brief Description of Drawings}

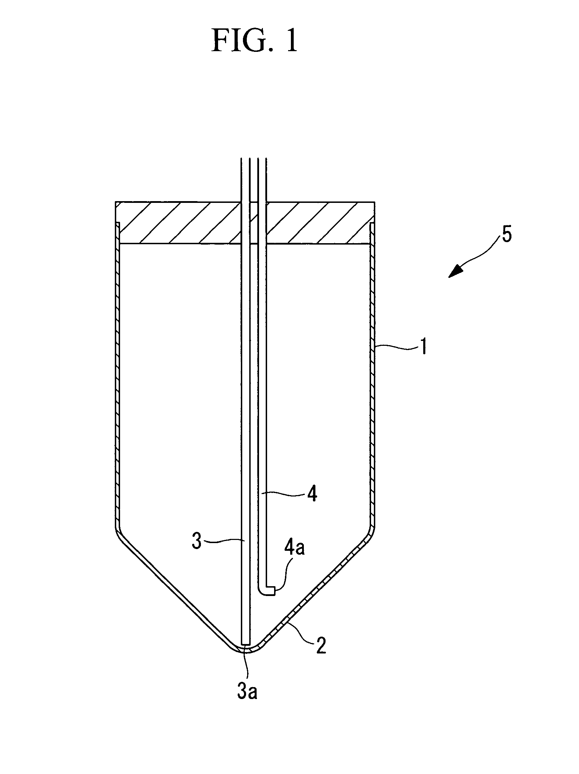

{Fig. 1}
   Fig. 1 is a longitudinal sectional view of a centrifuge container used in a cell washing method according to an embodiment of the present invention.
{Fig. 2}
   Fig. 2 is a flow chart illustrating the cell washing method of Fig. 1.
{Fig. 3}
   Fig. 3 is a graph illustrating the remaining enzyme concentration when washing is performed with centrifugal motion at different centrifugal speeds in comparison with when washing is performed without centrifugal motion.
{Fig. 4}
   Fig. 4 is a graph illustrating the number of cells alive in a collected pellet-shaped cell cluster measured with different discharge speeds of washing fluid.
{Fig. 5}
   Fig. 5 is a graph illustrating the number of cells alive in a collected pellet-shaped cell cluster measured with different centrifugal accelerations.

### {Description of Embodiments}

A cell washing method according to an embodiment of the present invention will be described below with reference to the drawings.
As illustrated in Fig. 1, the cell washing method according to this embodiment is performed using a centrifuge container 5 including a container main body 1 having a bottom part tapered toward the tip, a discharge suction tube 3 having a discharge suction port 3a disposed near the bottom part 2 inside the container main body 1, and a supernatant suction tube 4 having a suction port 4a disposed at a position away from the discharge suction port 3a in the axial direction.

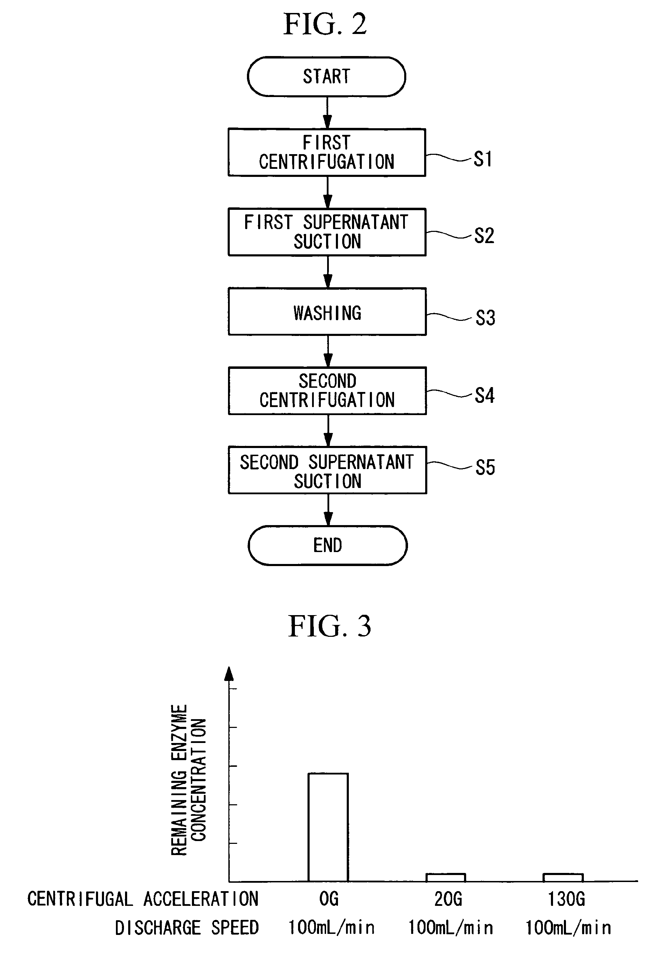

As illustrated in Fig. 2, the cell washing method includes a first centrifuging step S1 of centrifugally separating a cell suspension by specific gravity into a supernatant and a cell cluster by rotating the container main body 1, holding the cell suspension, in such a manner that the bottom part 2 faces outward in the radial direction; a first supernatant ejecting step (first supernatant sucking step) S2 of ejecting the supernatant centrifugally separated in the first centrifuging step S1 to the outside of the container main body 1 by suction through the suction port 4a of the supernatant suction tube 4; a washing step S3 of causing resuspension by discharging washing fluid from the discharge suction port 3a while rotating the container main body 1 from which the supernatant has been ejected; a second centrifuging step S4 of separating the supernatant and the cell cluster by centrifugally separating the cell suspension resuspended by supplying the washing fluid in the washing step S3; and a second supernatant ejecting step S5 of ejecting the supernatant centrifugally separated in the second centrifuging step (second supernatant sucking step) S4 to the outside of the container main body 1 by suction through the suction port 4a of the supernatant suction tube 4.

By centrifugally separating the cell suspension into the supernatant and the cell cluster in the first centrifuging step S1, unwanted components, such as digestive enzyme solution and dust, contained in the cell suspension are trapped between the cells and are formed into a pellet shape. In this state, in the supernatant ejecting step S2, by ejecting the supernatant from the container main body 1, the pellet-shaped cell cluster remains inside the container main body 1. Then, in the washing step S3, the washing fluid is supplied to the pellet-shaped cell cluster from the discharge suction port 3a of the discharge suction tube 3 facing the bottom part 2 to resuspend and wash the cell cluster such that the unwanted components trapped in the cell cluster are released.

In such a case, according to this embodiment, in the washing step S3, by discharging the washing fluid toward the bottom part 2 of the container main body 1 while rotating the container main body 1 in a centrifugal motion, the washing fluid can be supplied while collecting the cell cluster receiving the centrifugal force at the bottom part 2 of the container main body 1. In this way, the washing fluid can be supplied while retaining the pellet-shaped cell cluster at the bottom part 2 so that the pellet-shaped cell cluster is not scattered by the discharged washing fluid, and the pellet-shaped cell cluster can be more reliably broken down to release the unwanted components tapped inside.

Then, the cell suspension generated by breaking down the cell cluster in the washing step S3 is centrifugally separated into the supernatant and the pellet-shaped cell cluster in the second centrifuging step S4, and in the second supernatant ejecting step S5, a pellet-shaped cell cluster can be obtained again by ejecting the supernatant. The concentration of unwanted components contained in the cell cluster obtained in this way is sufficiently lower than those in the cell cluster obtained initially.

Fig. 3 illustrates the concentration of the digestive enzyme remaining inside the obtained pellet-shaped cell cluster when the washing fluid is discharged at a discharge speed of 100 mL/min from the discharge suction port 3a while centrifugally moving the container main body 1 at a centrifugal acceleration of 20G and 130G. Fig. 3 also illustrates, for comparison, the remaining digestive enzyme concentration of when the washing fluid is discharged at a discharge speed of 100 mL/min without centrifugal motion.

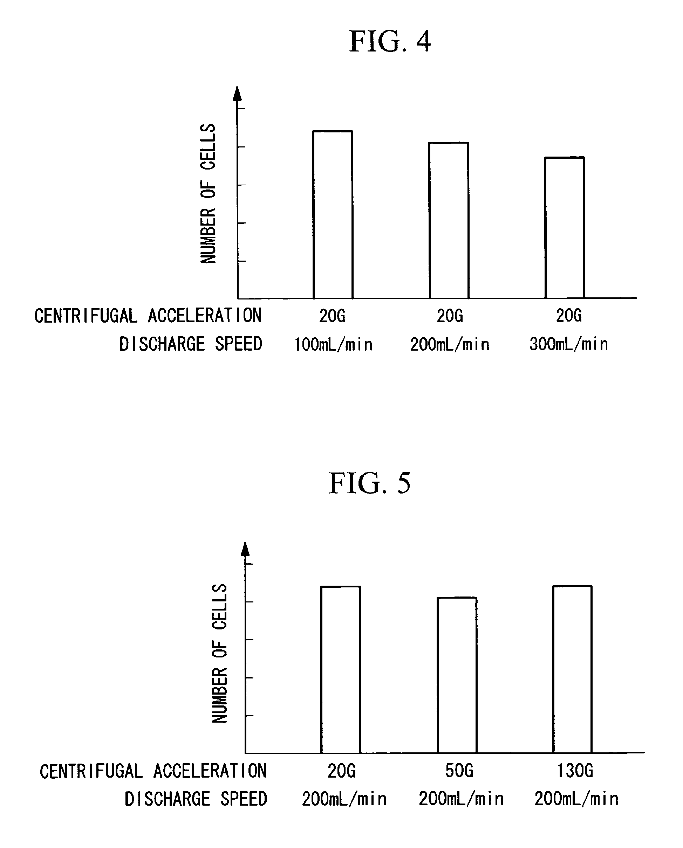

As illustrated in Fig. 3, regardless of the centrifugal acceleration, the remaining digestive enzyme concentration (U/mL) inside the pellet-shaped cell cluster obtained when the washing fluid is discharged to the cell cluster while centrifugally moving the container main body 1 decreases significantly compared with when the washing fluid is discharged without centrifugal motion.
As illustrated in Figs. 4 and 5, it is clear that the number of cells alive in the pellet-shaped cell cluster obtained as described above is acquired independently of the centrifugal acceleration and the supply speed of the washing fluid in the washing step. Fig. 4 illustrates the number of cells (cells) when the centrifugal acceleration is fixed at 20G and the discharge speed of the washing fluid is changed from 100 to 300 mL/min; and Fig. 5 illustrates the number of cells (cells) when the discharge speed is fixed at 200 mL/min and the centrifugal acceleration is changed from 20 to 130G.

In this way, according to the cell washing method of this embodiment, since a cell cluster having a sufficiently suppressed concentration of remaining unwanted components can be acquired, and the concentration of digestive enzymes, which are unwanted components, is low, there are advantages in that damage to the cells is prevented, and a cell cluster having high biological affinity can be acquired by removing unwanted components from a cell cluster to be implanted.
Furthermore, after resuspension by supplying a small amount of washing fluid from the discharge suction port 3a of the discharge suction tube 3, the cell suspension may be collected outside the container main body 1 from the discharge suction port 3a of the discharge suction tube 3.

### {Reference Signs List}

- 1: container main body (centrifuge container)
- 2: bottom part
- 3a: discharge port (discharge suction port)
- S1, S4: centrifuging step
- S2, S5: supernatant ejecting step (supernatant sucking step)
- S3: washing step

## Claims

1. A cell washing method comprising:
a centrifuging step of collecting a cell suspension in a centrifuge container having a washing fluid discharge port opposing a bottom part and centrifugally separating cells at the bottom part by rotating in such a manner that the bottom part faces outward in the radial direction;
a supernatant ejecting step of ejecting a supernatant centrifugally separated in the centrifuging step; and
a washing step of discharging washing fluid from the discharge port while rotating the centrifuge container while ejecting the supernatant in the supernatant ejecting step.

2. The cell washing method according to Claim 1, wherein the centrifuging step and the supernatant ejecting step are performed again after the cells are washed in the washing step.
